# EUROPEAN PATENT APPLICATION

(11) **EP 0 979 633 A1**
(43) Date of publication of application: **16.02.2000**
(21) Application number: 99202782.1
(22) Date of filing: 16.09.1996
(51) Int. Cl.: A61B 1/04

(54) **Video endoscope with side view optics**

(30) Priority: 15.09.1995 US 3802 P; 30.08.1996 US 708044
(62) Divisional of application: 96932255.1
(71) Applicant: Pinotage, LLC, Fayetteville, Arkansas 72701 (US)
(72) Inventor: Thomson, Robert Lee, Dallas, Texas 75243 (US)
(74) Representative: Britter, Keith Palmer

(57) **Abstract**

Image-sensing apparatus comprising: an elongate sheath (11;190); an image sensor (204) having an imaging axis (17) and disposed within the sheath (11;190); and a support member (7,9;178) which has a longitudinal axis (27), supports the image sensor (204) within the sheath (11;190) such that the imaging axis (17) can be oriented transversely to the longitudinal axis (27) of the support member (7,9;178), is at least partially disposed within the sheath (11;190) and is rotatable relative to the sheath (11;190).

## Description

This invention is related to an endoscope for use in interabdominal, interthoracic, and other surgical and diagnostic procedures on the human body.

Endoscopic surgery and diagnosis are considerably less invasive than the conventional procedures. This results in a lower mortality rate and minimizes both the patient's hospital stay and recovery time.

Conventional endoscopes include a rigid elongate member, a lens assembly, and an imaging device mounted either on or within the endoscope. Examples of such endoscopes are described in US Patents Nos. 4,697,210 (Toyota et al), 4,791,479 (Ogiu et al), and 4,989,586 (Furukawa).

Although a conventional endoscope can be constructed to have a wide field of view, the picture quality suffers. As a practical matter, the field of view of conventional endoscopes must be relatively narrow. As a result, a conventional endoscope must be positioned carefully at the beginning of a procedure, then held in position throughout the procedure, which generally requires the full-time attention of one member of the operating team. US Patent No. 5,351,678 (Clayton et al) addresses the initial positioning problems by providing an endoscope having a distal end which is offset from the endoscope's longitudinal axis. With the Clayton et al endoscope, the surgeon can easily change the area viewed by rotating the endoscope about its longitudinal axis. However, the Clayton et al endoscope must still be held in place throughout the procedure by a member of the operating team.

Generally, the prior art endoscopes comprise: an image-sensing device having an imaging axis and pivotally connected to and adjacent a distal end of an elongate support shaft, to allow the device to pivot and thereby move the imaging axis in a plane parallel to a longitudinal axis of the support shaft; and elevation means arranged to so-pivot the image-sensing device. The image-sensing device may be mounted in a camera housing, in which case, the camera housing is pivotally connected to and adjacent the distal end of the elongate support shaft, to allow the housing to pivot and thereby move the imaging axis in a plane parallel to the longitudinal axis of the support shaft.

The inventive endoscope is characterised in that it comprises: an elongate sheath within which the image-sensing device, and any associated camera housing, and at least a portion of the support shaft and elevation means are disposed and which has a longitudinal axis substantially coincident with the support shaft longitudinal axis; and azimuth means for rotating the support shaft about the longitudinal axis thereof, thereby rotating the support shaft relative to the longitudinal axis of the sheath.

An endoscope embodying the invention may include an imaging device housing a charge-coupled device ("CCD") and an associated lens mounted within a camera bore in the camera housing which may be pivotally mounted at the distal end of an elongate camera support. High intensity lights may also be mounted within bores in the camera housing that are coaxial with the camera bore and thus with the axis of the CCD.

Prior to use, the camera housing and camera support tube are inserted into a disposable sterile sheath. The distal portion of the sheath is then inserted into the patient through an incision in the patient. Electric stepper motors and associated components may be provided to move the camera housing, and thus the CCD, in elevation and azimuth.

The imaging device is preferably electrically connected to a control console. The control console may in turn be electrically connected to a display device and a control assembly. The display device displays the image received by the CCD and the control assembly allows the surgeon to control the elevation and azimuth of the camera housing.

The imaging device is easily aimed at the area of interest within the patient by the surgeon. In addition, the imaging device need not be held in position in the patient by a member of the operating team.

Embodiments of the inventive endoscope will now be described, by way of example with reference to the accompanying drawings, in which:
Figure 1 is a front view of a first embodiment of endoscope in accordance with the invention;
Figure 2 is a partially cutaway side view of the endoscope of Figure 1;
Figure 3 is a cutaway top view of a sheath cap taken through plane 3-3 in Figure 1;
Figure 4 is an enlarged cutaway side view of an upper housing and a lower portion of the endoscope;
Figure 5 is a cutaway top view of the upper housing taken through plane 5-5 in Figure 4;
Figure 6 is a cutaway top view of the lower portion of the endoscope taken through plane 6-6 in Figure 4;
Figure 7 is a block diagram of a first system for controlling the endoscope of Figure 1 and for displaying the images transmitted by the endoscope;
Figure 8 is a block diagram of a second control and display system for the endoscope of Figure 1;
Figure 9 is a cutaway side view of a second embodiment of endoscope in accordance with the invention;
Figure 10 is a front view of a camera housing of the endoscope shown in Figure 9; and
Figure 11 is a cutaway side view of the camera housing taken through plane 11-11 in Figure 10.

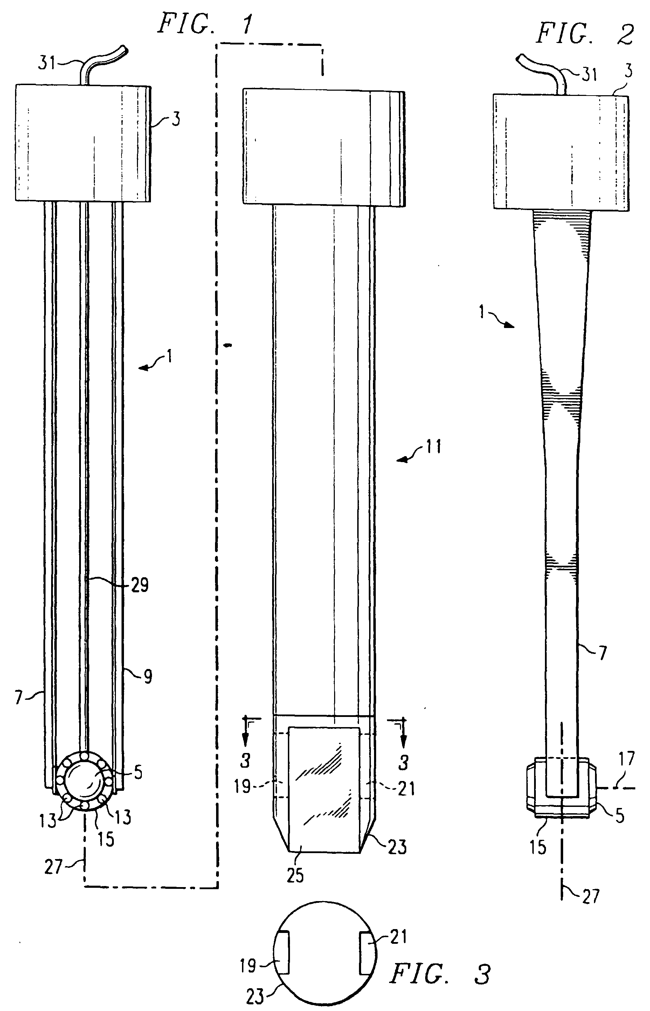

Figures 1-3 show a first embodiment of inventive endoscope for use in interabdominal, interthoracic, and other surgical and diagnostic procedures. The endoscope comprises an image-sensing device, indicated generally at 1, having an upper housing 3, a camera housing 5, and left and right camera housing supports 7, 9. Before use, the image-sensing device 1 is inserted into a sterile sheath 11. The device 1 and sheath 11 (collectively, called the "camera") are then inserted throughout an incision (not shown) into the patient's body (not shown). The camera is inserted so as to place the camera housing 5 in a position from which it can be pointed at the surgical site or the area to be diagnosed. The incision is sealed around the camera with a purse string stitch, thereby preventing leakage of CO₂ gas which is used to distend the patient's abdomen or chest during surgery or diagnosis.

In this embodiment, the sheath 11 is constructed of a medical-grade plastics material, is provided in a sterilized condition, and is intended to be disposed of after use. Alternatively, the sheath 11 can be constructed of a heat-resistant material in order to allow it to be sterilized using an autoclave and then reused. It will be appreciated that the sterile sheath 11 eliminates the need to sterilize the camera.

The camera housing 5 contains a charge-coupled device (CCD - not shown) and a zoom lens assembly (also not shown). A plurality of high intensity lights 13 are mounted within a light housing 15 which extends about the outer circumference of the camera housing 5. The lights 13 are aligned with the focal axis 17 of the CCD and provide illumination of the area at which the camera housing 5, and hence the CCD, is pointed.

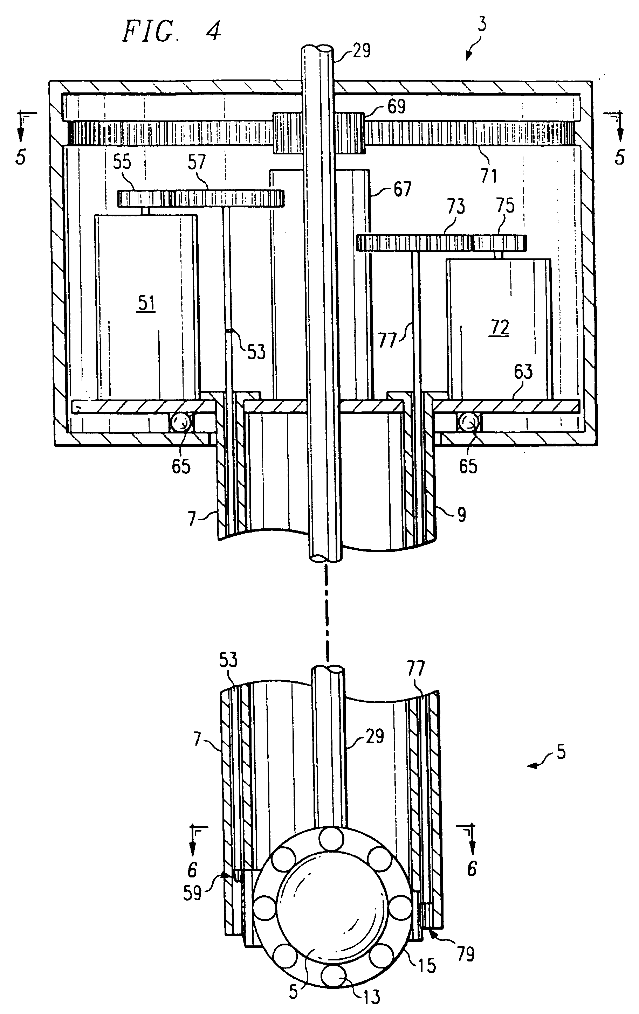

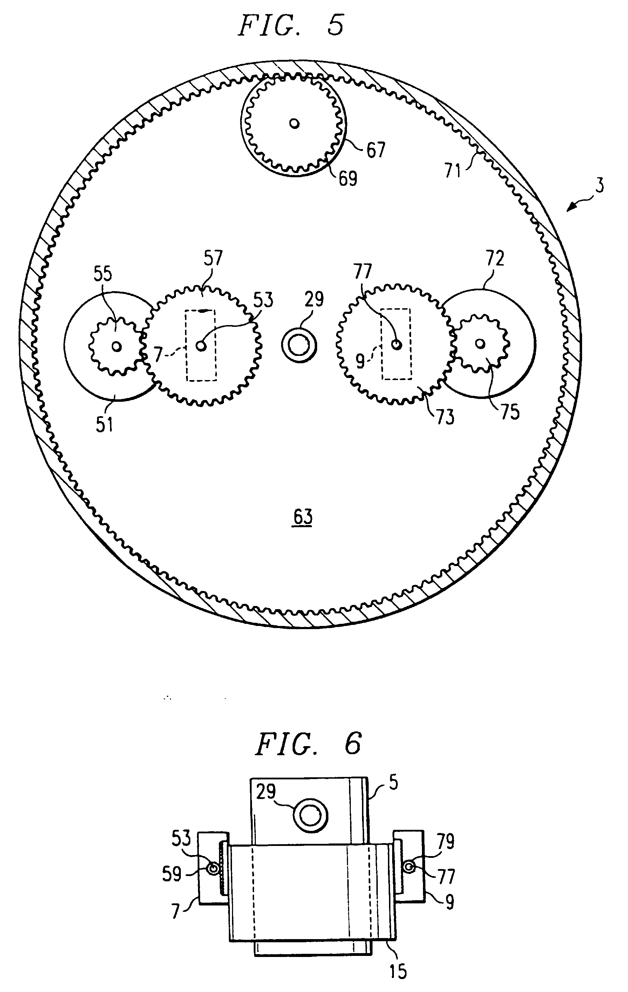

When the image-sensing device 1 is inserted in the sheath 11, the left and right camera housing supports 7, 9 engage complementary locking keys 19, 21 within a sheath cap 23. As a result, the camera housing 5 is locked into a position in which the CCD's focal axis 17 is aligned perpendicular to an optically-clear window 25. In addition, and as will be described below in connection with Figures 4-6, the locking keys 19, 21 cause the sheath cap 23 to rotate about the longitudinal axis 27 of the camera when the camera housing supports 7, 9 are rotated about that axis.

A camera cable 29 extends between the camera housing 5 and the upper housing 3 and contains conductors which carry the CCD's signals to the upper housing 3 and which supply electrical power to the CCD and lights 13. An endoscope cable 31 is provided to carry control signals and supply electrical power to the endoscope and to carry the CCD's signals to external processing and display devices (not shown).

The length of the camera housing supports 7, 9 and the length of the sheath 11 are varied to accommodate variation in the thickness of the abdominal walls of patients and to allow the camera to be used in thoracic surgery/diagnosis. Three lengths are provided: 7.62 cms, 15.24 cms and 27.94 cms ( 3, 6, and 11 inches) below the upper housing 3.

Referring now to Figures 4-6, an elevation motor 51 drives an elevation shaft 53 by means of gears 55, 57. The elevation shaft 53 extends downwardly through the hollow left camera support 7. A ring and pinion gear arrangement 59 at the lower end of the elevation shaft 53 transfers the rotary motion of the elevation shaft 53 to the camera housing 15, thereby causing the camera housing 15 to elevate or depress, depending on the direction of rotation of the elevation motor 51. In this embodiment of the invention, the camera housing 15 can be elevated 70 degrees above and depressed 90 degrees below a plane perpendicular to the longitudinal axis 27 of the camera and passing through the intersection of the axis 27 and the focal axis 17 of the camera.

The elevation motor 51 is mounted on a plate 63. The plate 63 is rotatably mounted within the upper housing 3 on a bearing 65. An azimuth motor 67 is also mounted on the plate 63 and drives an azimuth gear 69 which engages a housing gear 71 attached to the inner surface of the upper housing 3. When the azimuth motor 67 rotates, the plate 63 rotates within the upper housing 3. In this embodiment, the plate 63 rotates plus or minus 180 degrees in order to minimize the amount by which the camera cable 21 is twisted. 360 degree rotation can easily be achieved by using conventional slip rings.

A zoom/focus motor 72 drives gears 73, 75, which rotate a zoom/focus shaft 77. The zoom/focus shaft 77 extends downwardly through the hollow right camera support 9.

At the bottom of the focus shaft 77, a ring and pinion arrangement 79 transfers the rotary motion of the focus shaft 77 to a zoom lens mechanism (not shown) within the camera housing 5.

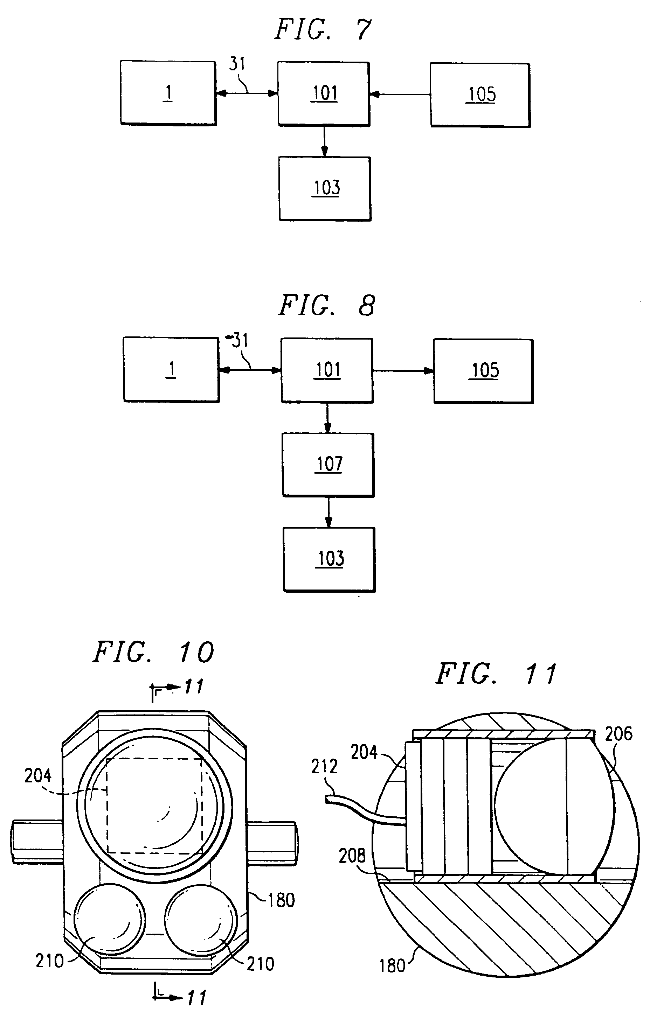

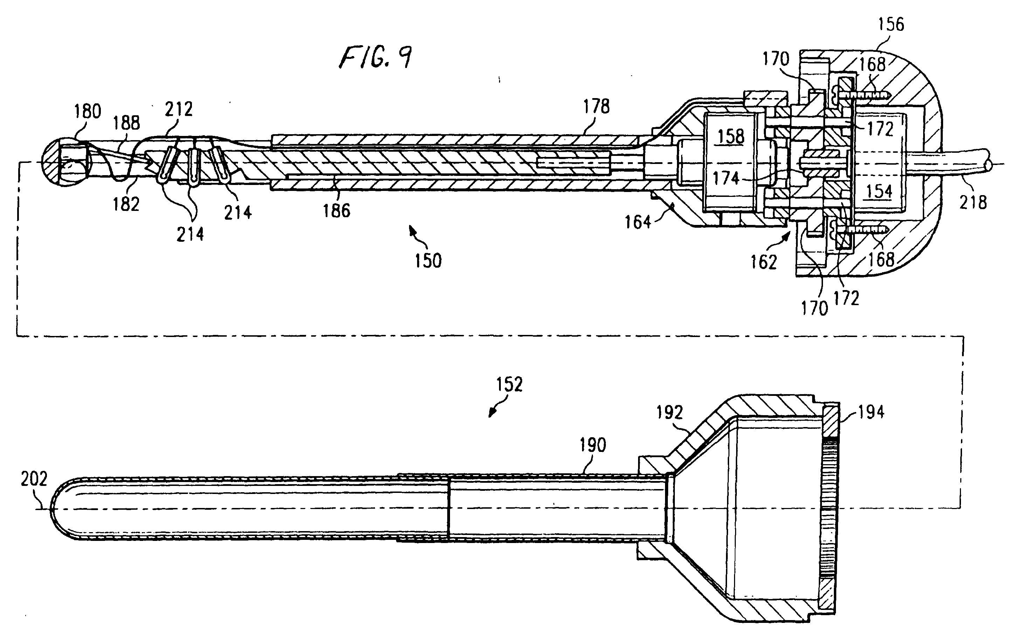

Referring now to Figure 7, the endoscope is connected to a control console 101 by means of the endoscope cable 31. Signals from the CCD of the image-sensing device 1 are amplified by circuits in the control console 101 and directed to a display device 203. In this embodiment of the invention, the display device 103 is a conventional television set.

A foot pedal control assembly 105 allows the surgeon (not shown) to control the endoscope. The foot pedal control assembly 105 includes four controls (not shown) for: (1) camera housing left and right (azimuth); (2) camera housing up and down (elevation); (3) zoom in and out; and (4) light intensity up and down. Signals from the foot pedal control assembly 105 are routed to the control console 101. Circuits (not shown) in the control console 103 convert the control assembly signals into signals which are suitable to control the endoscope, then route the converted signals to the endoscope.

In a modification of the invention shown in Figure 8, a computer 107 is interposed between the control console 101 and the display device 103. A plurality of computer programs contained in the computer 107 allow operating team personnel to manipulate and/or store the signals from the endoscope.

Figures 9-11 illustrate a second embodiment of endoscope in accordance with the invention.

Referring firstly to Figure 9, an endoscope comprises two major assemblies: a camera assembly 150 and a disposable sheath assembly 152.

In the camera assembly 150, a rotary stepper motor 154 is rigidly mounted in an upper housing 156. A linear stepper motor 158 and the distal end of a planetary gear assembly 162 are press fitted in a linear stepper motor housing 164. The proximal end of the planetary gear assembly 162 is attached to the upper housing 156 by screws 168.

Three planetary gears 170, of which only two are shown in Figure 9, are rotatably mounted on pins 172 within the planetary gear assembly 162. The rotary stepper motor 154 drives the planetary gears 170 through a sun gear 174.

The proximal end of a camera support tube 178 is press fitted in the linear stepper housing 164. A camera housing 180 is pivotally mounted between a pair of arms 182, of which only one is shown in Figure 9, that are integral with and extend from the distal end of the camera support tube 178. The linear stepper motor 158 acts through a pushrod 186 and a fork 188 to control the elevation of the camera housing 180.

The disposable sheath assembly 152 comprises a sheath 190, a sheath housing 192, and a ring gear 194. The distal portion of the sheath 190 is optically clear. The proximal end of the sheath 190 is adhesively attached within the distal end of the sheath housing 192. The ring gear 194 is adhesively attached within the proximal end of the sheath housing 192.

Prior to use, the camera assembly 150 is inserted into the sheath assembly 152 and the planet gears 170 engage the ring gear 194. As a result, when the rotary stepper motor 154 is actuated, the camera assembly 150 rotates in relation to the longitudinal axis 202 of the sheath assembly 152.

As is best shown in Figures 10 and 11, a CCD assembly 204 and a lens 206 are mounted within a camera bore 208 in the camera housing 180. A pair of high intensity lights 210 are mounted in bores that are coaxial with the camera bore 208.

A multi-conductor flexible cable 212 provides the necessary connections for the CCD assembly 204, for the camera housing lights 210, and for three high intensity lights 214 that are disposed in bores in the pushrod 186. The cable 212 extends from the camera housing 180 to the upper housing 156. In the upper housing 156, the cable 212 is combined with power and control wires (not shown) for the rotary stepper motor. 154 and the linear stepper motor 158 to form the camera assembly cable 218. The camera assembly cable 218 passes through an orifice 220 in the upper housing 152. As with the embodiment of the invention shown in Figures 1-8, the camera assembly cable 218 connects the camera assembly 150 to external display and control devices (not shown).

## Claims

1. Image-sensing apparatus comprising:
an elongate sheath (11;190);
an image sensor (204) having an imaging axis (17) and disposed within the sheath (11;190); and
a support member (7,9;178) which has a longitudinal axis (27), supports the image sensor (204) within the sheath (11;190) such that the imaging axis (17) can be oriented transversely to the longitudinal axis (27) of the support member (7,9;178), is at least partially disposed within the sheath (11;190) and is rotatable relative to the sheath (11;190).

2. Image-sensing apparatus as claimed in claim 1, wherein:
the support member (7,9;178) is elongate and has a distal end;
the sheath (11;190) has a longitudinal axis (202) which is substantially coincident with the longitudinal axis (27) of the support member (7,9;178); and
the apparatus further includes:
a camera housing (5;180) in which the image sensor (204) is mounted, which is disposed within the sheath (11;190) and which is connected pivotally adjacent the distal end of the support member (7,9;178) along an axis transverse to the longitudinal axis (27) of the support member (7,9;178) to allow the camera housing (5;180) to pivot and thereby move the imaging axis (17) in a plane parallel to the longitudinal axis (27) of the support member (7,9;178);
elevation means (51,53,55,57,59,158,186,188) which is arranged to pivot the camera housing (5;180) and of which at least a portion is disposed within the sheath (11;190); and
azimuth means (67,69,71;154,170,174) arranged to rotate the support member (7,9;178) about the longitudinal axis (27) of the support member (7,9;178), to rotate the support member (7,9;178) relative to the longitudinal axis (202) of the sheath (11;190).

3. Image-sensing apparatus as claimed in claim 1, wherein:
the support member (7,9;178) is elongate and has a distal end;
the sheath (11;190) has a longitudinal axis (202) which is substantially coincident with the longitudinal axis (27) of the support member (7,9;178);
the image sensor (204) is connected pivotally adjacent the distal end of the support member (7,9;178) along an axis transverse to the longitudinal axis (27) of the support member (7,9;178), to allow the image sensor (204) to pivot and thereby move the imaging axis (17) in a plane parallel to the longitudinal axis (27) of the support member (7,9;178); and the apparatus further includes:
elevation means (51,53,55,57,59;158,186,188) which is arranged to pivot the image sensor (204) and of which at least a portion is disposed within the sheath (11;190); and
azimuth means (67,69,71;154,170,174) arranged to rotate the support member (7,9;178) about the longitudinal axis (27) of the support member (7,9;178) to rotate the support member (7,9;178) and image sensor (204) relative to the longitudinal axis (202) of the sheath (11;190).

4. Image-sensing apparatus as claimed in claim 3 further comprising a camera housing (5;180) within which the image sensor (204) is mounted and which is attached pivotally to the support member (7,9;178) adjacent the distal end thereof

5. Image-sensing apparatus as claimed in any of claims 2 to 4, wherein said elevation means (51,53,55,57,59;158,186,188) comprises an elevation actuator (51;158) connected to the image sensor (204) by a gear-and-shaft assembly (53,55,57,59;186).

6. Image-sensing apparatus as claimed in claim 5 further comprising an elevation controller (101, 105) which is connected to the elevation actuator (51;158) and is adapted to control the position of the elevation actuator (51;158), thereby controlling the position of the imaging axis (17) in said plane.

7. Image-sensing apparatus as claimed in any of claims 2 to 4, wherein said elevation means (158,186,188) comprises a liner actuator (158) connected to the image sensor (204) by a pushrod (186).

8. Image-sensing apparatus as claimed in claim 7 comprising further an elevation controller (101,105) which is connected to the linear actuator (158) and is adapted to control the position of the linear actuator (158), thereby controlling the position of the imaging axis (17) in said plane.

9. Image-sensing apparatus as claimed in any of claims 2 to 8 comprising further an azimuth controller (101,105) which is connected to the azimuth means (67,69,71;154,170,174) and is adapted to control the position of the azimuth means (67,69,71;154,170,174) and, thereby, the position of the support member (7,9;178) and the image sensor (204) relative to the longitudinal axis (202) of the sheath (11;190).

10. Image-sensing apparatus as claimed in any of claims 2 to 9, wherein said azimuth means (67,69,71) comprises:
a plate (63) affixed to the support member (7,9) adjacent a proximal end thereof;
an azimuth actuator (67) including a gear (69) on an output shaft thereof and affixed to the plate (63);
an upper housing (3) disposed about the plate (63) and the azimuth actuator (67); and
a ring gear (71) affixed to an inner surface of the upper housing (3) and engaged with the azimuth actuator gear (69),
whereby, when the azimuth actuator (67) rotates the azimuth actuator gear (69), the plate (63) is rotated within the upper housing (3), thereby rotating the support member (7,9) relative to the longitudinal axis of the sheath (11).

11. Image-sensing apparatus as claimed in any of claims 2 to 9, wherein said azimuth means (154,170,174) comprises:
a ring gear (194) affixed to the sheath (190) adjacent a proximal end thereof;
an upper housing (156) to which the support member (178) is connected;
an azimuth actuator (154) affixed to the upper housing (156); and a gearset (162) connected to an output shaft of the azimuth actuator (154) and including at least one gear (170) engaged with the ring gear (194), whereby when the azimuth actuator (154) rotates, the upper housing (156) rotates relative to the longitudinal axis (202) of the sheath (190), thereby rotating the support member (178) and the image sensor (204) relative to the longitudinal axis (202) of the sheath (190).

12. Image-sensing apparatus as claimed in claim 1, wherein:
the support member (7,9;178) includes a proximal end and a distal end opposite the proximal end and supports the image sensor (204) near the distal end of the support member (7,9;178), such that the image sensor (204) can be moved relative to the sheath (11;190) about at least two axes of rotation; and
the image-sensing apparatus further includes at least one mechanical driver (51,72;158) located near the proximal end of the support member (7,9;178), to move the image sensor (204) about at least one of said at least two axes of rotation.

13. Image-sensing apparatus as claimed in claim 12, wherein:
said at least one mechanical driver (51,72; 158) includes an azimuth driver (72) and an elevation driver (51;158);
said at least two axes of rotation include an azimuth axis and an elevation axis;
the azimuth driver (72) is arranged to rotate the image sensor (204) about the azimuth axis; and
the elevation driver (51;158) is arranged to rotate the image sensor (204) about the elevation axis.

14. Image-sensing apparatus as claimed in claim 12 or 13 further comprising a controller (101,105) arranged to control said at least one mechanical driver (51,72;158).

15. Image-sensing apparatus as claimed in any of claims 1 and 12 to 14, wherein:
the support member (7,9;178) has a proximal end and a distal end opposite the proximal end and supports the image sensor (204) near the distal end of the support member (7,9;178), such that the image sensor (204) can be moved, relative to the sheath (11;190), about at least two axes of rotation; and
the image-sensing apparatus includes further at least one linkage system(53,55,57,59;73,75,77,79;186) arranged to transfer physical forces from the proximal end of the support member (7,9;178) to the distal end of the support member (7,9;178) so as to control movement of the image sensor (204) about at least one of said at least two axes of rotation.

16. Image-sensing apparatus as claimed in claim 15 wherein:
said at least one linkage system (53,55,57,59;73,75,77,79;186) includes an azimuth linkage system (73,75,77,79) and an elevation linkage system (53,55,57,59;186);
said at least two axes of rotation include an azimuth axis and an elevation axis,
the azimuth linkage system (73,75,77,79) is arranged to rotate the image sensor (204) about the azimuth axis; and
the elevation linkage system (53,55,57,59;186) is arranged to rotate the image sensor (204) about the elevation axis.

17. Image-sensing apparatus as claimed in claim 1 further comprising an azimuth driver (51;158) arranged to move the image sensor (204) to adjust an elevation of the imaging axis (17) relative to the longitudinal axis (27) of the support member(7,9;178).

18. Image-sensing apparatus as claimed in claim 17, wherein;
the support member (7,9;178) includes a distal end and a proximal end;
the image sensor (204) is supported by the support member (7,9;178) near the distal end of the support member (7,9;178); and
the azimuth driver (51;158) is located near the proximal end of the support member (7,9;178).

19. Image-sensing apparatus as claimed in claim 1 further comprising an azimuth driver (72) arranged to rotate the support member (7,9;178) relative to the sheath (11;190).

20. Image-sensing apparatus as claimed in claim 19, wherein:
the support member (7,9;178) includes a distal end and a proximal end;
the image sensor (204) is supported by the support member (7,9;178) near the distal end of the support member (7,9;178); and the azimuth driver (72) is located near the proximal end of the support member (7,9;178).

21. Image-sensing apparatus as claimed in any of claims 1 and 12 to 20, wherein:
the elongate sheath (11;190) has a longitudinal axis (202); and
the longitudinal axis (202) of the sheath (11;190) is substantially coincident with the longitudinal axis (27) of the support member (7,9;178).

22. Image-sensing apparatus as claimed in any of claims 1 and 12 to 21 further comprising a camera housing (5;180) supporting the image sensor (204) and connected pivotally to the support member (7,9;178).

23. Image-sensing apparatus as claimed in any of claims 1 and 12 to 22, wherein the support member (7,9;178) is elongate.

24. Image-sensing apparatus as claimed in any of claims 1 to 23, wherein the support member (7,9;178) is rigid.

25. Image-sensing apparatus as claimed in any of claims 1 to 24, wherein the image sensor (204) includes a solid-state device (204).

26. Image-sensing apparatus as claimed in any of claims 1 to 25, wherein the solid-state device (204) includes a charge coupled device (204).

27. Image-sensing apparatus as claimed in any of claims 1 to 26, wherein the elongate sheath (11;190) is rigid.

28. Image-sensing apparatus as claimed in any of claims 1 to 27, wherein the support member (7,9;178) is spaced from the sheath (11;190) throughout its range of motion.

29. Image-sensing apparatus as claimed in any of claims 1 to 28, wherein the image sensor (204) is supported by the support member (7,9;178), so that the imaging axis (17) of the image sensor (204) can pivot through a pivoting range about a pivot axis which maintains a constant relationship with respect to the support member (7,9;178) throughout the pivoting range of the imaging axis (17).

30. Image-sensing apparatus as claimed in any of claims 1 to 29, wherein the image sensor (204) is supported by the support member (7,9;178), so that the imaging axis (17) of the image sensor (204) can pivot about only one pivot axis.

31. Image-sensing apparatus as claimed in any of claims 1 to 30, wherein:
the imaging axis (17) of the image sensor (204) is oriented to view an area outside the sheath (11;190); and
the image-sensing apparatus includes further at least one light source (13;210,214) which is disposed within the sheath (11;190) to illuminate the viewing area outside the sheath (11;190) and which converts energy from a non-light energy source into light.
